# EUROPÄISCHE PATENTSCHRIFT

(11) **EP 0 752 710 B1**
(45) Veröffentlichungstag und Bekanntmachung des Hinweises auf die Patenterteilung: **10.11.1999**
(21) Anmeldenummer: 96110721.6
(22) Anmeldetag: 03.07.1996
(51) Int. Cl.: G21G 1/06, A61K 51/10

(54) **Verfahren zur Erzeugung von Actinium-225 aus Radium-226**
Process for producing actinium-225 from radium-226
Procédé pour produire de l'actinium-225 à partir du radium-226

(30) Priorität: 03.07.1995 LU 88637
(43) Veröffentlichungstag der Anmeldung: 08.01.1997
(73) Patentinhaber: EUROPÄISCHE ATOMGEMEINSCHAFT (EURATOM), 2920 Luxembourg (LU)
(72) Erfinder: Koch, Lothar, 76356 Weingarten (DE); Fuger, Jean, 75045 Wössingen (DE); van Geel, Jacques, 76275 Ettlingen-Oberweier (DE)
(74) Vertreter: Weinmiller, Jürgen

(56) Entgegenhaltungen:
- EP-A- 0 443 479
- WO-A-90/15625
- CHEMICAL ABSTRACTS, vol. 115, no. 12, 23.September 1991 Columbus, Ohio, US; abstract no. 11595100d, BEYER ET AL: "PREPARATION AND CHEMICAL SEPARATION OF ACTINIUM-225." Seite 641; Spalte L; XP002009430 & ZENTRALINST. KERNFORSCH. , ROSSENDORF Bd. ZFK, Nr. 711, DRESDEN, Seiten 17 - 20
- DATABASE INIS INTERNATIONAL ATOMIC ENERGY AGENCY (IAEA), VIENNA, AT AN: 21(17):66714, BEYER ET AL: "COMPARISON OF THE BIODISTRIBUTION OF 225AC AND RADIOLANTHANIDES AS CITRATES COMPLEXES" XP002009431 & ISOTOPENPRAXIS Bd. 26, Nr. 3, DDR, Seiten 111 - 114

## Beschreibung

Die Erfindung bezieht sich auf ein Verfahren zur Erzeugung von Actinium-225 aus Radium-226 mit Hilfe einer von einer Kernstrahlung ausgelösten Kernreaktion.

Ein solches Verfahren ist z.B. aus der EP-0 443 479-B1 bekannt. Gemäß diesem Verfahren wird das Radium-226 in einem thermischen Neutronenfluß eines Kernreaktors bestrahlt, wobei u.a. Thorium-229 entsteht. Der Thoriumanteil wird dann chemisch isoliert und daraus wird das entstehende Radium-225 abgetrennt. Schließlich wird aus dem Radium das einwachsende Actinium-225 isoliert.

Radiotherapeutische Methoden zur lokalen Bekämpfung von Krebserkrankungen (Metastasen) gewinnen mit Fortschritten in der Molekularbiologie immer mehr an Bedeutung. Im Prinzip werden dabei kurzlebige alphastrahlende Nuklide in monoklonale Antikörper eingebaut, die, in den Körper des Patienten gebracht, von den malignen Zellen bevorzugt aufgenommen werden und diese durch intensive Bestrahlung mit sehr kurzer Reichweite zerstören. An das Radionuklid werden dabei besondere Anforderungen gestellt: Es muß sich für die Konjugation an einen geeigneten Antikörper binden lassen, muß eine kurze Halbwertzeit haben (in der Größenordnung von einigen Stunden) und seine Zerfallsprodukte müssen chemisch und radiologisch weitgehend unschädlich sein.

Unter den möglichen Kandidaten für ein derartiges Radionuklid spielen Actinium-225 und Wismut eine hervorragende Rolle, und zwar bezüglich Wismut entweder das Isotop Bi-212 (Halbwertzeit 60,6 Minuten) oder das Isotop Bi-213 (Halbwertzeit 47 Minuten). Die Herstellung von Bi-212 für medizinische Zwecke wurde in der Zeitschrift Ing. J. Nucl. Med. Biol. 9 (1982), Seite 83 beschrieben. Dieses Isotop hat allerdings den Nachteil, daß als Folgeprodukt ein gamma-aktives Thalliumisotop auftritt, das zu einer unerwünschten Strahlenbelastung des Patienten führt.

Dies gilt zwar nicht für Bi-213, jedoch ist dieses Isotop nicht in ausreichenden Mengen verfügbar und kann auch bisher nicht mit vertretbarem Aufwand in diesen Mengen aus U-233 hergestellt werden. Dieses Uranisotop U-233 bildet über mehrere Zerfallsschritte Actinium-225 und dieses schließlich Bi-213.

Der Nachteil dieses bekannten Verfahrens liegt in der unerwünscht großen Verunreinigung des Actinium-225 mit anderen Actinium-Isotopen oder anderen Folgeprodukten der Zerfallsreihen von Thorium-232, Uran-238 und Uran-235.

Aufgabe der Erfindung ist es also, ein Verfahren der obengenannten Art anzugeben, bei dem das als Basisprodukt für die Therapie wichtige Isotop Ac-225 weniger stark verunreinigt ist.

Diese Aufgabe wird durch die in den unabhängigen Ansprüchen definierten Verfahren gelöst. Bezüglich von Merkmalen bevorzugter Ausführungsformen der Erfindung wird auf die abhängigen Ansprüche verwiesen.

Nachstehend wird die Erfindung anhand zweier bevorzugter Ausführungsbeispiele und der beiliegenden Zeichnungen näher erläutert.

Figur 1 zeigt einen Ausschnitt aus der Nuklidkarte mit den für das erfindungsgemäße Verfahren wichtigen Isotopen der Elemente Ra und Ac.

Figur 2 zeigt die natürliche Zerfallskette von Ac-225 zu dem therapeutisch wichtigen Bi-213.

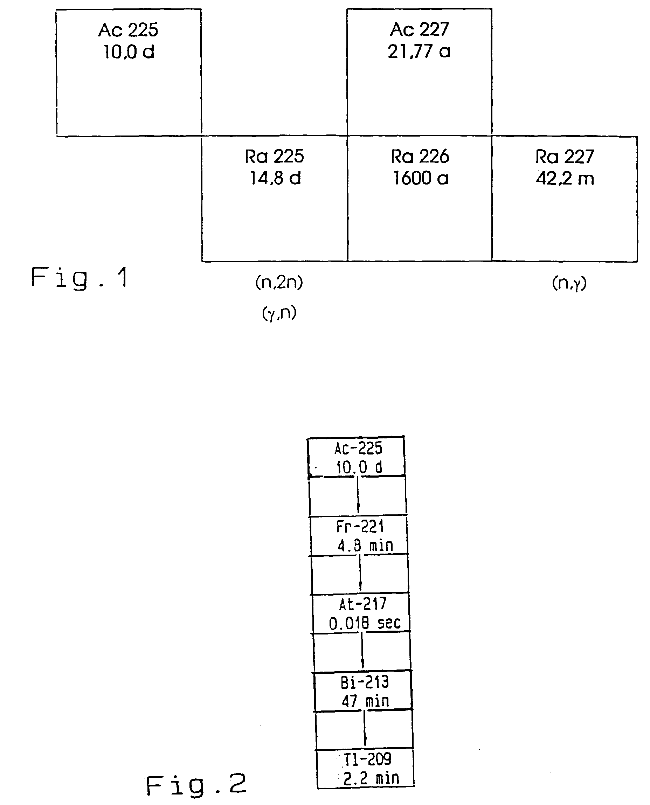

Ausgangspunkt des Verfahrens ist Radium-226. Man erzeugt ein Target aus diesem Material und setzt es in einem "schnellen Brüter" einem Fluß von schnellen Neutronen aus. Dabei ergibt sich durch eine (n,2n)-Kernreaktion Radium-225 sowie durch eine (n,γ)-Kernreaktion Radium-227. Wie Figur 1 zeigt, unterscheiden sich die Halbwertzeiten dieser beiden Radioisotope stark. Man nützt diesen Umstand aus und läßt das Target nach der Bestrahlung soweit abklingen, daß nach einer anschließenden chemischen Abtrennung des Actinium-Gemisches mangels Ra-227 kaum mehr unerwünschte Actinium-Isotope nachgebildet werden.

Nun kann aus dem Gemisch von Ra-226 (Target), Ra-225 und dem sich neu bildenden Ac-225 erneut chemisch das Ac-225 abgetrennt werden, das dann für therapeutische Zwecke, u.a. auch zur Bildung von Wismut-213 gemäß der natürlichen Zerfallskette, die in Figur 2 gezeigt ist, konfektioniert wird.

Im Rahmen der Erfindung kann die oben erwähnte Bestrahlung durch einen schnellen Neutronenfluß auch in einer Spallationsquelle erfolgen. Weiter kann das Radium-225 auch durch Bestrahlung eines Radium-226-Targets mit einer hochenergetischen γ-Strahlung unmittelbar erzeugt werden, wobei dann eine (γ, n)-Kernreaktion in Frage kommt und praktisch kein störendes Radium-227 entsteht. Dabei entfällt also das Abklingen des Targets nach der Bestrahlung vor der ersten chemischen Isolierung des sich bildenden Actiniums. Diese γ-Strahlung kann insbesondere von der Bremsstrahlung beschleunigter Elektronen in einem Elektronenbeschleuniger gebildet werden.

Die Erfindung erlaubt eine dem Bedarf leicht anzupassende Erzeugung von sauberem Ac-225 und seiner therapeutisch wichtigen Zerfallsprodukte.

## Patentansprüche

1. Verfahren zur Erzeugung von Actinium-225 aus Radium-226 mit Hilfe einer von einer Kernstrahlung ausgelösten Kernreaktion, dadurch gekennzeichnet, daß ein Target aus Radium-226 einer Bestrahlung mit hochenergetischen Neutronen ausgesetzt wird, bei der durch eine (n,2n)-Reaktion Ra-225 entsteht, daß nach einer Wartezeit, in der durch eine (n,γ)-Reaktion entstandenes Ra-227 größtenteils zerfällt, die inzwischen durch β-Zerfall gebildeten Actiniumisotope aus dem Targetmaterial chemisch abgetrennt werden und daß aus dem verbleibenden Targetmaterial nochmals neu gebildetes Actinium chemisch abgetrennt wird, bei dem es sich fast ausschließlich um das Isotop Ac-225 handelt.

2. Verfahren nach Anspruch 1, dadurch gekennzeichnet, daß die Bestrahlung in einem "schnellen" Reaktor mit hochenergetischen Neutronen erfolgt.

3. Verfahren nach Anspruch 1, dadurch gekennzeichnet, daß die Bestrahlung mittels hochenergetischer Neutronen erfolgt, die aus einer Spallationsquelle stammen.

4. Verfahren zur Erzeugung von Actinium-225 aus Radium-226 mit Hilfe einer von einer Kernstrahlung ausgelösten Kernreaktion, dadurch gekennzeichnet, daß ein Target aus Radium-226 einer Bestrahlung mit hochenergetischen γ-Strahlen ausgesetzt wird, bei der durch eine (γ,n)-Kernreaktion Ra-225 entsteht, worauf das sich daraus durch β-Zerfall bildende Ac-225 chemisch aus dem Trägermaterial abgetrennt wird.

5. Verfahren nach Anspruch 4, dadurch gekennzeichnet, daß die Bestrahlung in der Bremsstrahlung eines Elektronenbeschleunigers erfolgt.

## Claims

1. A method for producing actinium 225 from radium 226 by means of a nuclear reaction caused by an exposure to nuclear radiation, characterized in that a target made of radium 226 is exposed to a radiation with high energy neutrons, whereby Ra-225 is obtained by a reaction of the type (n,2n), that after a period of time, during which most of the Ra-227 decays which might have been generated by a reaction of the type (n,γ), the actinium isotopes obtained in the meantime by a β-type decay are chemically separated from the target material and that finally from the remaining target material newly produced actinium is again chemically separated, this actinium being almost exclusively the isotope Ac-225.

2. A method according to claim 1, characterized in that the radiation exposure takes place in a "fast" reactor with high energy neutrons.

3. A method according to claim 1, characterized in that the radiation uses high energy neutrons from a spallation source.

4. A method for producing actinium 225 from radium 226 by means of a nuclear reaction caused by an exposure to a nuclear radiation, characterized in that a target of radium 226 is exposed to a radiation with high energy gamma rays whereby Ac-225 is produced via a nuclear reaction of the (γ,n) type, where-upon Ac-225 obtained therefrom via a β-decay is separated chemically from the target material.

5. A method according to claim 4, characterized in that the radiation exposure takes place in the bremsstrahlung of an electron accelerator.

## Revendications

1. Procédé pour produire de l'actinium 225 (Ac-225) à partir de radium 226 (Ra-226) à l'aide d'une réaction nucléaire provoquée par un rayonnement nucléaire, caractérisé en ce qu'une cible en radium 226 est exposée à un rayonnement par des neutrons très énergétiques, cequi donne naissance à du Ra-225 par une réaction du type (n,2n), en ce qu'après une période d'attente, pendant laquelle du Ra-227, qui, le cas échéant, a été produit parallèlement par une réaction du type (n,γ), se désintègre pour la plus grande partie, les isotopes d'actinium obtenus entre-temps par désintégration du type γ sont isolés chimiquement des matériaux de la cible et qu'enfin de l'actinium nouvellement produit à partir des matériaux restant de la cible est isolé chimiquement, cet actinium étant presqu'exclusivement l'isotope Ac-225.

2. Procédé selon la revendication 1, caractérisé en ce que l'irradiation se fait dans un réacteur "rapide" avec des neutrons très énergétiques.

3. Procédé selon la revendication 1, caractérisé en ce que l'irradiation se fait à l'aide de neutrons très énergétiques résultant d'une source du type spallation.

4. Procédé pour produire de l'actinium 225 à partir de radium 226 à l'aide d'une réaction nucléaire provoquée par un rayonnement nucléaire, caractérisé en ce qu'une cible en radium 226 est exposée à un rayonnement gamma très énergétique ce qui donne naissance à du Ra-225 par une réaction nucléaire du type (γ,n) et qu'ensuite de l'Ac-225 qui s'en forme par une désintégration du type β est separé chimiquement des matériaux de la cible.

5. Procédé selon la revendication 4, caractérisé en ce que l'irradiation se fait dans la bremsstrahlung d'un accélérateur d'électrons.
